# EUROPEAN PATENT SPECIFICATION

(11) **EP 4 196 633 B1**
(45) Date of publication and mention of the grant of the patent: **03.09.2025**
(21) Application number: 21851413.1
(22) Date of filing: 08.07.2021
(51) Int. Cl.: D06F 58/45, A61L 2/04, A61L 2/10, A61L 2/24, D06F 58/20, D06F 103/08, D06F 103/32, D06F 103/38, D06F 103/50, D06F 105/38

(54) **A LAUNDRY DRYER COMPRISING A UV LIGHT SOURCE**
WÄSCHETROCKNER MIT UV-LICHTQUELLE
SÈCHE-LINGE COMPRENANT UNE SOURCE DE LUMIÈRE UV

(30) Priority: 30.07.2020 TR 202012159
(43) Date of publication of application: 21.06.2023
(73) Proprietor: Arçelik Anonim Sirketi, 34445 Istanbul (TR)
(72) Inventor: ÇALICIOGLU, Hüseyin Mert, 34445 Beyoglu/Istanbul (TR); SIR, Gökhan, 34445 Beyoglu/Istanbul (TR); ARSLAN, Sergen Erkan, 34445 Beyoglu/Istanbul (TR); ÇALISIR, Kübra, 34445 Beyoglu/Istanbul (TR); ACIR, Aysin, 34445 Beyoglu/Istanbul (TR)
(86) International application number: PCT/TR2021/050703
(87) International publication number: WO 2022/025847

(56) References cited:
- EP-A2- 2 703 540
- CN-A- 103 485 142
- CN-A- 106 245 294
- CN-A- 106 555 320
- US-A1- 2009 249 837
- US-A1- 2019 127 905

## Description

The present invention relates to a laundry dryer comprising a UV light source.

It is aimed to eliminate viruses and bacteria on the laundry while the same is treated and dried in laundry dryers. Most of the viruses and bacteria cannot survive at temperatures above 56° C. Additional, UV light sources are used to eliminate viruses and bacteria in the laundry dryers. However, since the economic life of the UV light sources is not very long, said components are not activated during the entire drying process. In state of the art embodiments, generally after the laundry is detected to be dry, the UV light source is activated for a while so as to provide sterilization. Since the UV light sources cannot provide the same rate of radiation at every temperature, an efficient sterilization cannot be performed when the UV light source is operated after the drying process.

In the Patent Application No. CN106245294, a laundry dryer comprising a UV light source is disclosed.

In the Patent Application No CN103485142, a laundry dryer comprising a ultraviolet lamp for disinfecting clothes in a rotatable drum is disclosed, wherein the laundry dryer comprises further an airflow circulation passage which provides the delivery of drying air into the drum, a heat pump with a compressor and a refrigerant cycle, a temperature sensor disposed in the airflow circulation passage to measure the temperature of air drawn from the drum, and a further temperature sensor arranged at the outlet of the condenser of the heat pump.

In the Patent Application No US2009249837, a washing and drying machine comprising an irradiating light source is disclosed.

The aim of the present invention is the realization of a laundry dryer with improved sterilization performance.

The laundry dryer of the present invention is defined in claim 1 and comprises a body; a drum which is disposed in the body; an air circulation duct which provides delivery of air into the drum; a compressor which is disposed in the body and which provides a refrigerant cycle; a first temperature sensor which is disposed on the compressor and which measures a temperature of the compressor; a second temperature sensor which is disposed in the air circulation duct and which measures a temperature of the air drawn from the drum; and
a UV light source which is disposed in the body and which emits UV light into the drum. The light source can be UV-A, UV-B or UV-C. In a preferred version of the present invention, the UV light source is a UV-C light source.

The laundry dryer of the present invention further comprises a control unit which is configured to operate the UV light source when a temperature value received from the second temperature sensor is above 40⁰ C and turns off the same when said temperature is above 56⁰ C if a difference between a temperature value received from the first temperature sensor and a temperature value received from the second temperature sensor is above a first threshold value predetermined by the producer and if a temperature value received from the second temperature sensor is above a second threshold value predetermined by the producer after the compressor is operated for a first period of time predetermined by the producer. After the compressor is operated, the temperature values received from the first temperature sensor and the second temperature sensor start to increase. After approximately 2,000 seconds, the compressor reaches the range where the same operates in steady state. For 2,000 seconds, the temperature of the compressor and the temperature of the air drawn from the drum increase. When the ambient temperature is the same room temperature, the difference between the temperature value received from the first temperature sensor and the temperature value received from the second temperature sensor is above the first threshold value Moreover, the temperature value received from the second temperature sensor is above the second threshold value. When the ambient temperature is approximately the same as the room temperature, the temperature received from the second temperature sensor reaches 40° C in approximately 2,700 seconds. If the compressor continues operating, the temperature received from the second temperature sensor reaches 56⁰ C in approximately 6,000 seconds.

In an embodiment of the present invention, the control unit operates the UV light source if a difference between a temperature value received from the first temperature sensor and a temperature value received from the second temperature sensor is below the first threshold value and if a temperature value received from the second temperature sensor is below the second threshold value, and turns off the UV light source when a temperature value received from the second temperature sensor exceeds 56⁰ C. When the ambient temperature is approximately 5⁰ C, the difference between the temperature value received from the first temperature sensor and the temperature value received from the second temperature sensor is below the first threshold value Moreover, the temperature value received from the second temperature sensor is below the second threshold value.

When the ambient temperature is approximately 5⁰ C, the temperature of the drying air in the drum reaches 40⁰ C in a period of time longer than 6,000 seconds. Therefore, when the ambient temperature is detected to be low thanks to the measurements made after the first period of time, the control unit operates the UV light source immediately after the end of the first period of time. Thus, by operating the UV light source for a longer period of time at a low ambient temperature, the viruses and bacteria on the laundry are eliminated.

In an embodiment of the present invention, the laundry dryer comprises a humidity sensor. When a value received from the humidity sensor falls below a predetermined value, the control unit decides that the laundry in the drum is dry, and the drying process is terminated. At the end of the drying process, the control unit turns off the compressor and the UV light source.

In an embodiment of the present invention, the control unit does not operate the UV light source if a temperature value received from the second temperature sensor is below 4⁰ C. The sterilization effect and the radiation power of the UV light source significantly decrease at temperatures under 4⁰ C. Consequently, energy efficiency is provided.

In an embodiment of the present invention, the first threshold value is 25⁰ C. In an embodiment of the present invention, the second threshold value is 30⁰ C.

By means of the present invention, a laundry dryer is realized, wherein the sterilization performance is improved by means of a control unit which operates the UV light source at ranges where the same can be efficiently operated in terms of sterilization.

The advantages of the laundry dryer of the present invention will be disclosed with the detailed description of the embodiments with reference to the accompanying figure, where:

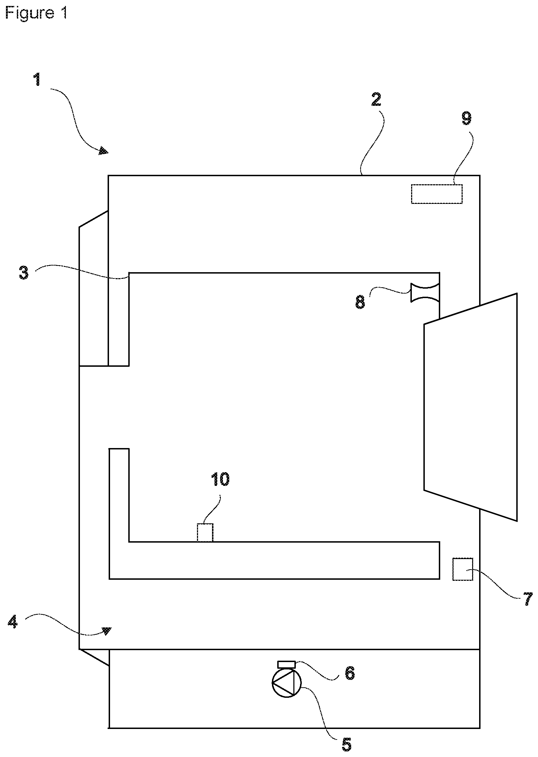

Figure 1 - is the schematic view of the laundry dryer in an embodiment of the present invention.

The following numerals are referred to in the description of the present invention:
- 1.: Laundry dryer
- 2.: Body
- 3.: Drum
- 4.: Air circulation duct
- 5.: Compressor
- 6.: First temperature sensor
- 7.: Second temperature sensor
- 8.: UV light source
- 9.: Control unit
- 10.: Humidity sensor

The laundry dryer (1) comprises a body (2); a drum (3) which is disposed in the body (2) and wherein the laundry is loaded; an air circulation duct (4) which provides the delivery of the drying air into the drum (3); a compressor (5) which is disposed in the body (2) and which provides the refrigerant cycle; a first temperature sensor (6) which is disposed on the compressor (5) and which measures the temperature of the compressor (5); a second temperature sensor (7) which is disposed in the air circulation duct (4) and which measures the temperature of the air drawn from the drum (3); and a UV light source (8) which is disposed in the body (2) and which emits UV light into the drum (3). The second temperature sensor (7) is positioned in the vicinity of the air outlet of the drum (3). In the preferred version of the present invention, the second temperature sensor (7) is disposed behind the filter (not shown in figures) which retains the fibers breaking off from the laundry during the drying process. The UV light source (8) is positioned on the part, facing the inner volume of the drum (3), of the front bearing which rotatably supports the drum (3).

The laundry dryer (1) comprises a control unit (9) which operates the UV light source (8) when the temperature value received from the second temperature sensor (7) is above 40⁰ C and turns off the same when said temperature is above 56⁰ C if the difference between the temperature value received from the first temperature sensor (6) and the temperature value received from the second temperature sensor (7) is above a first threshold value predetermined by the producer and if the temperature value received from the second temperature sensor (7) is above a second threshold value predetermined by the producer after the compressor (5) is operated for a first period of time predetermined by the producer. The UV light source (8) provides a higher amount of radiation as the ambient temperature increases. For example, when operated at 4⁰ C, the UV light source (8) radiates at 100 microwatt/cm² while the same radiates at approximately 125 microwatt/cm² at 20⁰ C. The amount of radiation exceeds 140 microwatt/cm² at temperatures above 40° C, ensuring a higher light intensity. At temperatures above 56⁰ C, most of bacteria and viruses cannot survive due to high heat. Therefore, the UV light source (8) is not required to be operated at temperatures above 56⁰ C. In the laundry dryers (1) wherein the ambient temperature is close to the room temperature, the drying air can be delivered into the drum (3) at a high temperature in a short time. However, in the laundry dryers (1) wherein the ambient temperature is approximately 5⁰ C, it takes time to heat the drying air to be delivered into the drum (3) to high temperatures. The ambient temperature is detected by means of the data received from the first temperature sensor (6) which is provided on the compressor (5) and which measures the temperature of the body of the compressor (5), and the second temperature sensor (7) which measures the temperature of the air drawn from the drum (3). To this end, first the compressor (5) is operated for a first period of time. The compressor (5) is required to be operated for a period of time to reach the efficient operation range. This period of time is approximately between 1,900 seconds and 2,100 seconds in the laundry dryers (1). In a preferred version of the embodiment, the first period of time is 2,000 seconds. The increase rate of the temperature of the compressor (5) varies depending on the temperature of the environment where the laundry dryer (1) is installed. After the compressor (5) is operated, the temperature of the air delivered into the drum (3) also increases. As the ambient temperature increases, the increase rate of the temperature of the air drawn from the drum (3) decreases. For example, in the laundry dryer (1) operated at an ambient temperature of 23° C, the value received from the second temperature sensor (7) is 35⁰ C at the mark of 2,000 seconds while in the laundry dryer (1) operated at an ambient temperature of 5⁰ C, the value received from the second temperature sensor (7) is 25⁰ C at the mark of 2,000 seconds. It is determined that the ambient temperature is almost the same as the room temperature (23⁰ C) if the difference between the temperature value received from the first temperature sensor (6) and the temperature value received from the second temperature sensor (7) is above a first threshold value predetermined by the producer and if the temperature value received from the second temperature sensor (7) is above a second threshold value predetermined by the producer. In the laundry dryer (1) operated under conditions where the ambient temperature is almost the same as the room temperature, the temperature of the air delivered into the drum (3) can be increased in a short time. In this case, the UV light source (8) is operated at temperatures between 40° C and 56⁰ C where the efficiency thereof is high, the laundry loaded into the drum (3) is enabled to be sterilized. When the value received from the second temperature sensor (7) exceeds 40⁰ C, the control unit (9) operates the UV light source (8). When the value received from the second temperature sensor (7) falls below 40⁰ C or exceeds 56⁰ C, the control unit (9) turns off the UV light source (8).

In another embodiment of the present invention, the laundry dryer (1) comprises the control unit (9) which operates the UV light source (8) if the difference between the temperature value received from the first temperature sensor (6) and the temperature value received from the second temperature sensor (7) is below the first threshold value and if the temperature value received from the second temperature sensor (7) is below the second threshold value, and which turns off the UV light source (8) when the temperature value received from the second temperature sensor (7) exceeds 56⁰ C. When the laundry dryer (1) is operated at a low ambient temperature, the increase rate of the temperature of the compressor (5) decreases a little while the increase rate of the temperature of the air drawn from the drum (3) increases. In this case, after the compressor (5) is operated for approximately 2,000 seconds, the difference between the temperature value received from the first temperature sensor (7) and the temperature value received from the second temperature sensor (7) is below the first threshold value.

Moreover, in the low ambient temperature, after the first period of time elapses upon the operation of the compressor (5), the value received from the second temperature sensor (7) is below the second threshold value predetermined by the producer. Since the temperature of the air drawn from the drum (3) reaches 40⁰ C in a long time when the laundry dryer (1) is operated at low ambient temperature, the control unit (9) immediately operates the UV light source (8) if, at the end of the first period of time, the difference between the temperature value received from the first temperature sensor (6) and the temperature value received from the second temperature sensor (7) is below the first threshold value and if the temperature value received from the second temperature sensor (7) is below the second threshold value. When the temperature value received from the second temperature sensor (7) exceeds 56⁰ C, the control unit (9) turns off the UV light source (8). In a version of the present invention, if the temperature value received from the second temperature sensor (7) never exceeds 56⁰ C during the program selected via the control panel, the control unit (9) turns off the UV light source (8) at the end of the program.

In another embodiment of the present invention, the laundry dryer (1) comprises a humidity sensor (10) which is provided in the body (2) and which measures the humidity of the laundry, and the control unit (9) which turns off the UV light source (8) if the value received from the humidity sensor (10) falls below a value predetermined by the producer. The humidity sensor (10) is positioned in the body (2) so as to contact the laundry. It is decided that the laundry is dry when the value received from the humidity sensor (10) falls below a value predetermined by the producer. The drying process is ended and the UV light source (8) is turned off after the control unit (9) detects that the laundry is dry. Thus, the user is prevented from being exposed to UV light while he/she takes the laundry out of the drum (3) after the drying process is ended.

**In** an embodiment of the present invention, the laundry dryer (1) comprises the control unit (9) which does not allow the operation of the UV light source (8) if the temperature value received from the second temperature sensor (7) is below 4⁰ C. The UV light source (8) operates at low levels of radiation at temperatures under 4⁰ C, and the sterilization effect thereof becomes significantly inefficient. Therefore, the control unit (9) does not operate the UV light source (8) if the temperature value received from the second temperature sensor (7) is below 4⁰ C. Thus, the energy saving of the laundry dryer (1) is increased.

**In** an embodiment of the present invention, the first threshold value is between 22⁰ C and 28⁰ C. **In** the laundry dryer (1) operated in an environment at the room temperature, the difference between the temperature value received from the first temperature sensor (6) and the temperature value received from the second temperature sensor (7) at the end of the first period of time is approximately 30⁰ C. In the laundry dryer (1) operated in an environment with an ambient temperature of approximately 5⁰ C, the difference between the temperature value received from the first temperature sensor (6) and the temperature value received from the second temperature sensor (7) at the end of the first period of time is approximately 20⁰ C. In a preferred embodiment of the present invention, the first threshold value is 25⁰ C.

In an embodiment of the present invention, the second threshold value is between 27⁰ C and 33⁰ C. In the laundry dryer (1) operated in an environment at the room temperature, the temperature value received from the second temperature sensor (7) at the end of the first period of time is 35⁰ C while in the laundry dryer (1) operated in an environment with an ambient temperature of approximately 5⁰ C, the temperature value received from the second temperature sensor (7) at the end of the first period of time is 25⁰ C. In a preferred embodiment of the present invention, the second threshold value is 30⁰ C.

By means of the present invention, a laundry dryer (1) is realized, comprising a control unit (9) which enables the UV light source (8) to provide radiation at the temperatures where the same is most efficient in terms of virus and bacteria elimination performance depending on the ambient conditions.

## Claims

1. A laundry dryer (1) comprising a body (2); a drum (3) which is disposed in the body (2) and wherein laundry is loaded; an air circulation duct (4) which provides delivery of drying air into the drum (3); a compressor (5) which is disposed in the body (2) and which provides a refrigerant cycle; a first temperature sensor (6) which is disposed on the compressor (5) and which measures a temperature of the compressor (5); a second temperature sensor (7) which is disposed in the air circulation duct (4) and which measures a temperature of the air drawn from the drum (3); and a UV light source (8) which is disposed in the body (2) and which emits UV light into the drum (3), the laundry dryer (1) further comprising:
- a control unit (9) configured to operate the UV light source (8) when a temperature value received from the second temperature sensor (7) is above 40⁰ C and to turn off the same when said temperature value is above 56⁰ C if a difference between a temperature value received from the first temperature sensor (6) and a temperature value received from the second temperature sensor (7) is above a first threshold value predetermined by the producer and if a temperature value received from the second temperature sensor (7) is above a second threshold value predetermined by the producer after the compressor (5) is operated for a first period of time predetermined by the producer.

2. A laundry dryer (1) as in Claim 1, wherein the control unit (9) is configured to operate the UV light source (8) if a difference between a temperature value received from the first temperature sensor (6) and a temperature value received from the second temperature sensor (7) is below the first threshold value and if a temperature value received from the second temperature sensor (7) is below the second threshold value, and wherein the control unit (9) is configured to turn off the UV light source (8) when a temperature value received from the second temperature sensor (7) exceeds 56⁰ C.

3. A laundry dryer (1) as in Claim 1, further comprising a humidity sensor (10) which is provided in the body (2) and which is configured to measure a humidity of the laundry, and wherein the control unit (9) is configured to turn off the UV light source (8) if a value received from the humidity sensor (10) falls below a predetermined value.

4. A laundry dryer (1) as in any one of the above claims, wherein the control unit (9) is configured to not allow the operation of the UV light source (8) if a temperature value received from the second temperature sensor (7) is below 4⁰ C.

5. A laundry dryer (1) as in any one of the above claims, wherein the first threshold value is between 22° C and 28⁰ C.

6. A laundry dryer (1) as in any one of the above claims, wherein the second threshold value is between 27⁰ C and 30⁰ C.

## Patentansprüche

1. Ein Wäschetrockner (1) umfasst einen Körper (2); eine Trommel (3), die in dem Körper (2) angeordnet ist und in die Wäsche geladen wird; eine Luftzirkulationsleitung (4), die für die Zufuhr von Trocknungsluft in die Trommel (3) sorgt; einen Kompressor (5), der in dem Körper (2) angeordnet ist und der einen Kühlmittelkreislauf bereitstellt; einen ersten Temperatursensor (6), der an dem Kompressor (5) angeordnet ist und der eine Temperatur des Kompressors (5) misst; einen zweiten Temperatursensor (7), der in der Luftzirkulationsleitung (4) angeordnet ist und der eine Temperatur der von der Trommel (3) angesaugten Luft misst; und eine UV-Lichtquelle (8), die in dem Körper (2) angeordnet ist und die UV-Licht in die Trommel (3) emittiert, wobei der Wäschetrockner (1) weiterhin umfasst:
- eine Steuereinheit (9), die so konfiguriert ist, dass sie die UV-Lichtquelle (8) betreibt, wenn ein von dem zweiten Temperatursensor (7) empfangener Temperaturwert über 400 C liegt, und diese ausschaltet, wenn der Temperaturwert über 560 C liegt, wenn eine Differenz zwischen einem von dem ersten Temperatursensor (6) empfangenen Temperaturwert und einem von dem zweiten Temperatursensor (7) empfangenen Temperaturwert über einem ersten, von dem Hersteller vorgegebenen Schwellenwert liegt, und wenn ein von dem zweiten Temperatursensor (7) empfangener Temperaturwert über einem zweiten, von dem Hersteller vorgegebenen Schwellenwert liegt, nachdem der Kompressor (5) für eine erste, von dem Hersteller vorgegebene Zeitspanne betrieben wurde.

2. Ein Wäschetrockner (1), wie in Anspruch 1 aufgeführt, wobei die Steuereinheit (9) konfiguriert ist, um die UV-Lichtquelle (8) zu betreiben, wenn eine Differenz zwischen einem Temperaturwert, der von dem ersten Temperatursensor (6) empfangen wird, und einem Temperaturwert, der von dem zweiten Temperatursensor (7) empfangen wird, unter dem ersten Schwellenwert liegt, und wenn ein Temperaturwert, der von dem zweiten Temperatursensor (7) empfangen wird, unter dem zweiten Schwellenwert liegt, und wobei die Steuereinheit (9) konfiguriert ist, um die UV-Lichtquelle (8) auszuschalten, wenn ein Temperaturwert, der von dem zweiten Temperatursensor (7) empfangen wird, 56⁰ C überschreitet.

3. Ein Wäschetrockner (1), wie in Anspruch 1 aufgeführt, der weiterhin einen Feuchtigkeitssensor (10) umfasst, der in dem Körper (2) vorgesehen ist und der konfiguriert ist, um eine Feuchtigkeit der Wäsche zu messen, und wobei die Steuereinheit (9) konfiguriert ist, um die UV-Lichtquelle (8) auszuschalten, wenn ein von dem Feuchtigkeitssensor (10) empfangener Wert unter einen vorbestimmten Wert fällt.

4. Ein Wäschetrockner (1), wie in einem der vorherigen Ansprüchen aufgeführt, wobei die Steuereinheit (9) so konfiguriert ist, dass sie den Betrieb der UV-Lichtquelle (8) nicht zulässt, wenn ein von dem zweiten Temperatursensor (7) empfangener Temperaturwert unter 4⁰ C liegt

5. Ein Wäschetrockner (1), wie in einem der vorherigen Ansprüchen aufgeführt, wobei der erste Schwellenwert zwischen 22⁰ C und 28⁰ C liegt.

6. Ein Wäschetrockner (1), wie in einem der vorherigen Ansprüchen aufgeführt, wobei der zweite Schwellenwert zwischen 27⁰ C und 30⁰ C liegt.

## Revendications

1. Un sèche-linge (1) comprenant un corps (2) ; un tambour (3) qui est disposé dans le corps (2) et dans lequel le linge est chargé ; un conduit de circulation d'air (4) qui permet l'acheminement de l'air de séchage dans le tambour (3) ; un compresseur (5) qui est disposé dans le corps (2) et qui assure cycle de réfrigération ; un premier capteur de température (6) qui est disposé sur le compresseur (5) et qui mesure la température du compresseur (5) ; un second capteur de température (7) qui est disposé dans le conduit de circulation d'air (4) et qui mesure la température de l'air aspiré depuis le tambour (3) ; et une source de lumière UV (8) qui est disposée dans le corps (2) et qui émet de la lumière UV dans le tambour (3), le sèche-linge (1) comprenant en outre :
- une unité de commande (9) configurée pour faire fonctionner la source de lumière UV (8) lorsque la valeur de température reçue du second capteur de température (7) est supérieure à 40 °C et pour l'éteindre lorsque ladite valeur de température dépasse 56 °C si une différence entre la valeur de température reçue du premier capteur de température (6) et la valeur de température reçue du second capteur de température (7) est supérieure à une première valeur seuil prédéfinie par le fabricant et si la valeur de température reçue du second capteur de température (7) est supérieure à une seconde valeur seuil prédéfinie par le fabricant après que le compresseur (5) a fonctionné pendant une première période prédéfinie par le fabricant.

2. Un sèche-linge (1) selon la Revendication 1, dans lequel l'unité de commande (9) est configurée pour faire fonctionner la source de lumière UV (8) si une différence entre la valeur de température reçue du premier capteur de température (6) et la valeur de température reçue du second capteur de température (7) est inférieure à la première valeur seuil, et si la valeur de température reçue du second capteur de température (7) est inférieure à la seconde valeur seuil, et dans lequel l'unité de commande (9) est configurée pour éteindre la source de lumière UV (8) lorsque la valeur de température reçue du second capteur de température (7) dépasse 56 °C.

3. Un sèche-linge (1) selon la Revendication 1, comprenant en outre un capteur d'humidité (10) qui est disposé dans le corps (2) et qui est configuré pour mesurer l'humidité du linge, et dans lequel l'unité de commande (9) est configurée pour éteindre la source de lumière UV (8) si la valeur reçue du capteur d'humidité (10) descend en dessous d'une valeur prédéterminée.

4. Un sèche-linge (1) selon l'une quelconque des revendications précédentes, dans lequel l'unité de commande (9) est configurée pour empêcher le fonctionnement de la source de lumière UV (8) si la valeur de température reçue du second capteur de température (7) est inférieure à 40 °C.

5. Un sèche-linge (1) selon l'une quelconque des revendications précédentes, dans lequel la première valeur seuil est comprise entre 22 °C et 28 °C.

6. Un sèche-linge (1) selon l'une quelconque des revendications précédentes, dans lequel la seconde valeur seuil est comprise entre 27 °C et 30 °C.
